# EUROPEAN PATENT SPECIFICATION

(11) **EP 1 838 688 B1**
(45) Date of publication and mention of the grant of the patent: **02.07.2014**
(21) Application number: 05853500.6
(22) Date of filing: 09.12.2005
(51) Int. Cl.: C07D 307/46, C07D 307/42

(54) **PREPARATION OF 2,5-(HYDROXYMETHYL)FURALDEHYDE (HMF) FROM FRUCTOSE**
HERSTELLUNG VON 2,5-(HYDROXYMETHYL)FURALDEHYD (HMF) AUS FRUCTOSE
PROCÉDÉ POUR LA PRÉPARATION DE 2,5-(HYDROXYMÉTHYL)FURALDEHYDE (HMF) A PARTIR DE FRUCTOSE

(30) Priority: 10.12.2004 US 635406 P; 02.03.2005 US 70063
(43) Date of publication of application: 03.10.2007
(60) Divisional application: 10005763.7
(73) Proprietor: Archer-Daniels-Midland Company, Decatur, IL 62526 (US)
(72) Inventor: SANBORN, Alexandra, J., Lincoln, IL 62656-5600 (US)
(74) Representative: Von Kreisler Selting Werner - Partnerschaft von Patentanwälten und Rechtsanwälten mbB
(86) International application number: PCT/US2005/044598
(87) International publication number: WO 2006/063220

(56) References cited:
- WO-A-03/024947
- FR-A- 2 663 933
- FR-A- 2 664 273
- FR-A- 2 669 635
- FR-A- 2 669 636
- FR-A- 2 670 209
- JP-A- 55 053 280
- US-A- 2 929 823
- US-A- 3 483 228
- DATABASE CA [Online] CHEMICAL ABSTRACTS SERVICE, COLUMBUS, OHIO, US; 2003, BICKER, M. ET AL: "Dehydration of fructose to 5-hydroxymethylfurfural in sub- and supercritical acetone" XP002381410 retrieved from STN Database accession no. 2003:274299 & GREEN CHEMISTRY , 5(2), 280-284 CODEN: GRCHFJ; ISSN: 1463-9262, 2003,
- DATABASE CA [Online] CHEMICAL ABSTRACTS SERVICE, COLUMBUS, OHIO, US; 1960, BONNER, T. G. ET AL: "The iodine-catalyzed conversion of sucrose into 5-( hydroxymethyl ) furfuraldehyde" XP002381436 retrieved from STN Database accession no. 1960:62655 & JOURNAL OF THE CHEMICAL SOCIETY 787-91 CODEN: JCSOA9; ISSN: 0368-1769, 1960,
- DATABASE CA [Online] CHEMICAL ABSTRACTS SERVICE, COLUMBUS, OHIO, US; 28 April 2004 (2004-04-28), YADAV, GANAPATI D. ET AL: "Selectivity engineering in conversion of sugars into 5-hydroxymethylfurfuraldehyde" XP002398123 retrieved from STN Database accession no. 2004:222109 & ABSTRACTS OF PAPERS, 227TH ACS NATIONAL MEETING, ANAHEIM, CA, UNITED STATES, MARCH 28-APRIL 1, 2004 , CELL-063 PUBLISHER: AMERICAN CHEMICAL SOCIETY, WASHINGTON, D. C. CODEN: 69FGKM, 2004,
- DATABASE CA [Online] CHEMICAL ABSTRACTS SERVICE, COLUMBUS, OHIO, US; 2002, RIBEIRO, MARCELO L. ET AL: "Synthesis of 2,5-furandicarboxylic acid from fructose : a suitable precursor for biopolymers" XP002398124 retrieved from STN Database accession no. 2003:999470 & NATURAL POLYMERS AND COMPOSITES IV, PROCEEDINGS FROM THE INTERNATIONAL SYMPOSIUM ON NATURAL POLYMERS AND COMPOSITES, 4TH, SAO PEDRO, BRAZIL, SEPT. 1-4, 2002 , 192-197. EDITOR(S): CAPPARELLI MATTOSO, LUIZ HENRIQUE; LEAO, ALCIDES; FROLLINI, ELISABETE., 2002,
- DATABASE CA [Online] CHEMICAL ABSTRACTS SERVICE, COLUMBUS, OHIO, US; 2000, KROGER, MARTIN ET AL: "A new approach for the production of 2,5-furandicarboxylic acid by in situ oxidation of 5-hydroxymethylfurfural starting from fructose" XP002398125 retrieved from STN Database accession no. 2000:763008 & TOPICS IN CATALYSIS , 13(3), 237-242 CODEN: TOCAFI; ISSN: 1022-5528, 2000,
- DATABASE CA [Online] CHEMICAL ABSTRACTS SERVICE, COLUMBUS, OHIO, US; 2000, BENVENUTI, F. ET AL: "Heterogeneous zirconium and titanium catalysts for the selective synthesis of 5-hydroxymethyl-2-furaldehyde from carbohydrates" XP002398126 retrieved from STN Database accession no. 2000:83722 & APPLIED CATALYSIS, A: GENERAL , 193(1,2), 147-153 CODEN: ACAGE4; ISSN: 0926-860X, 2000,
- DATABASE CA [Online] CHEMICAL ABSTRACTS SERVICE, COLUMBUS, OHIO, US; 1995, GRIN, S. A. ET AL: "Character of acid catalysis in the dehydration of fructose with formation of 5-( hydroxymethyl ) furfural" XP002398127 retrieved from STN Database accession no. 1995:173907 & KHIMICHESKAYA FIZIKA , 13(5), 113-18 CODEN: KHFID9; ISSN: 0207-401X, 1994,
- DATABASE CA [Online] CHEMICAL ABSTRACTS SERVICE, COLUMBUS, OHIO, US; 1981, NAKAMURA, YOSHIO: "Preparation of 5-( hydroxymethyl ) furfural by selective dehydration of D- fructose" XP002398128 retrieved from STN Database accession no. 1981:156646 & NOGUCHI KENKYUSHO JIHO , (23), 25-38 CODEN: NOGUAR; ISSN: 0369-5131, 1980,
- MUSAU ET AL: "The Preparation of 5-Hydroxymethyl-2-Furaldehyde (HMF) from D-Fructose in the Presence of DMSO" BIOMASS, vol. 13, 1987, pages 67-74, XP002398112
- FAYET ET AL: "Nouvelle méthode de préparation du 5-hydroxyméthyl-2-furaldéhyde par action de sels d'ammonium ou d'immonium sur öles mono-, oligo- et poly-saccharides, accés direct aux 5-halogénométthyl-2-furaldéhydes" CARBOHYDRATE RESEARCH, vol. 122, 1983, pages 59-68, XP002398113

## Description

### Field Of The Invention

Improved methods of producing chemical compounds are included herein. The dehydration reaction of common carbohydrates to form commercially important compounds, furan derivatives, and methods of optimizing the reactions to efficiently synthesize the products, as well as improved methods of purification are included herein.

### Background Of The Invention

2,5-(Hydroxymethyl)furaldehyde, also known as 2,5-(hydroxymethyl)furfural (HMF), has many important industrial and commercial applications, largely due to its many functional groups and ability to serve as a precursor in many polymerization reactions. HMF, for example, is a suitable starting source for the formation of various furan monomers required for the preparation of non-petroleum-derived polymeric materials. HMF, as well as other 2,5-disubstituted furanic derivatives, also has great potential for use in the field of intermediate chemicals from regrowing resources. Also due to its various functionalities, HMF may be used to produce a wide range of products, including, but not limited to, polymers, solvents, surfactants, pharmaceuticals, and plant protecting agents. HMF is shown in the structure below:

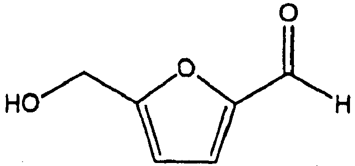

The use of HMF and other furfural derivatives may be compared with the use of corresponding benzene-based macromolecular compounds. In order to be cost-effective and compete in this market, HMF must be able to be produced at competitive prices. The production of HMF has been studied for years, but an efficient and cost-effective method of producing HMF in high yields has yet to be found. HMF is primarily produced from the dehydration reaction of a carbohydrate compound, particularly monosaccharides, including glucose and fructose. Complications arise from the rehydration of HMF after the dehydration occurs, which often yields the by-products of levulinic acid, and formic acid. Another competing side reaction is the polymerization of HMF and/or fructose to form humin polymers.

Hexoses are the preferred carbohydrate source from which HMF is formed. Fructose is the preferred hexose used for the dehydration reaction to form HMF. This is in part because fructose has been shown to be more amendable to the dehydration reaction to a form HMF. Fructose is shown by the structures below:

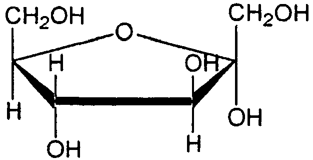

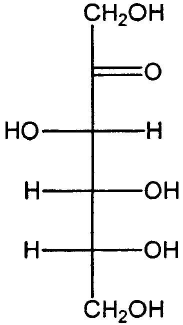

Fructose however, is more expensive than other hexoses, such as glucose (dextrose), and maltose, for example. Early processes and procedures for the production of HMF concentrated on the use of crystalline fructose, but its widespread use is prevented by its high cost. Other sources of fructose, including high-fructose corn syrup (HFCS), have been used to produce HMF and other furan derivatives. Szmant and Chundury used high fructose corn syrup as a starting material in forming HMF, as disclosed in a 1981 article in J. Chem. Tech. Biotechnol., 31, (pgs. 135-145). Szmant uses a variety of carbohydrates as starting material, but designs reaction conditions specific to each fructose source. Szmant, for example, uses a boron trifluoride catalyst (BF₃ Et₂O) with DMSO as a solvent in the conversion of HFCS to HMF, but utilizes different catalyst/solvent combinations with different starting materials. Use of BF₃ Et₂O as a catalyst is not economically practical since it cannot be recovered and re-used. Furthermore, Szmant requires the use of a Pluronic emulsifier to suppress foaming. Szmant also requires bubbling of nitrogen to suppress oxidation. Still further, Szmant requires the use of DMSO as a solvent, which is not easily separable from the HMF product, and therefore creates difficulties with product recovery. It is very desirable, therefore, to develop an industrially practicable process for producing HMF in high purity.

U.S. Patent No. 6,706,900 to Grushin et al. (Grushin '900) also discloses the dehydration of fructose in the form of high-fructose corn syrup, to form HMF as an intermediate; but this process is performed in the context of forming diformylfuran, also known as 2,5-dicarboxaldehyde (DFF). The reaction proceeds in an aqueous environment, and the HMF that is formed is not isolated from the reaction mixture, but rather is directly converted to DFF without an isolation step. The reaction conditions of Grushin '900 are therefore not constrained by considerations of product yields of HMF, as it is formed as an intermediate that is not isolated as a product. More importantly from a practical commercial standpoint, Grushin '900 is not constrained by considerations of isolating HMF from the product mixture. An efficient method for producing HMF in desirable yields and sufficiently high purity from a natural and industrially convenient fructose source that may include other mixed carbohydrates has yet to be found.

Water has in the past been used as a solvent of choice in dehydration reactions forming HMF because of the solubility of fructose in water. Aqueous conditions, however, have proven to deleteriously affect the dehydration reaction of fructose to HMF in a variety of ways. Aqueous conditions have led to decreased yield of HMF as low selectivity for the dehydration reaction has been demonstrated. Furthermore, solvation of protons in water highly reduces the catalytic activity for the dehydration reaction. Low selectivity of the dehydration reaction simultaneously leads to increased polymerization reactions and humin formation, which also interfere with the synthesis of HMF.

In an attempt to solve such problems associated with aqueous systems, one proposed solution involves an improvement by simultaneously extracting HMF after the dehydration reaction. A similar attempt to improve yields involves the adsorption of HMF on activated carbon. The key factor in these processes is a rapid removal of HMF from the acidic medium in which it is formed. However, these systems generally suffer from high dilution or partially irreversible adsorption of HMF.

In another attempt to solve the problems of aqueous systems, an organic solvent may be added to the aqueous solution, such as, for example, butanol or dioxane. Such systems, however, present a difficulty in that rehydration of HMF is common and ether formation of HMF occurs with the solvent if alcohols are employed. High yields of HMF, therefore, were not found with the addition of these organic solvents. In a further attempt to provide an adequate solvent system, aqueous solvent mixtures and anhydrous organic solvents have also been employed to ensure favorable reaction conditions. Examples of anhydrous organic solvents used include dimethylformamide, acetonitrile, dimethylsulfoxide, and polyethylene glycol.

Dimethylsulfoxide (DMSO), for example, has been extensively studied and employed as a solvent in the dehydration reaction to form HMF. Improved yields of HMF have been reached with ion exchangers or boron trifluoride etherate as a catalyst, and even without any catalyst. DMSO presents a problem, however, in that recovery of HMF from the solvent is difficult.

Furthermore, although dehydration reactions performed in solvents with high boiling points, such as dimethylsulfoxide and dimethylformamide, have produced improved yields, the use of such solvents is cost-prohibitive, and additionally poses significant health and environmental risks in their use. Still further, purification of the product via distillation has not proven effective for a variety of reasons. First of all, on long exposure to temperatures at which the desired product can be distilled, HMF and impurities associated with the synthetic mixture tend to be unstable and form tarry degradation products. Because of this heat instability, a falling film vacuum still must be used. Even in use with such an apparatus however, resinous solids form on the heating surface causing a stalling in the rotor, and the frequent shutdown resulting therefrom makes the operation inefficient.

Catalysts may also be used to promote the dehydration reaction. Some commonly used catalysts include cheap inorganic acids, such as H₂SO₄, H₃PO₄, HCl, and organic acids such as oxalic acid, levulinic acid, and p-toluene sulfonic acid. These acid catalysts are utilized in dissolved form, and as a result pose significant difficulties in their regeneration and reuse, and in their disposal. In order to avoid these problems, solid sulfonic acid catalysts have also been used. Solid acid resins, however, are limited in use by the formation of deactivating humin polymers on their surfaces under conditions taught by others. Other catalysts, such as boron trifluoride etherate, can also be used. Metals, such as Zn, Al, Cr, Ti, Th, Zr, and V can be used as ions, salts, or complexes as catalysts. Such use has not brought improved results, however, as yields of HMF have continued to be low. Ion exchange catalysts have also been used, but have also delivered low HMF yields under conditions taught by others, and further limit the reaction temperature to under 130°C.

WO 03/024947, FR 2 669 635, US 2,929,823, FR 2 664 273 and FR 2 669 636 relate to processes for the preparation of hydroxymethylfurfural from fructose or other carbohydrate sources.

Bicker et al., Green chemistry, 2003, 5(2), 280 to 284 describe the dehydration of fructose in sub- and supercritical acetone.

Bonner et al., Journal of the Chemical Society, 1960, pages 787 to 791 disclose the conversion of the fructose moiety in sucrose to hydroxymethylfuraldehyde.

FR 2 663 933; JP 55-053280; US 3,483,228; Yadav et al., Abstract of Papers, 227th ACS National Meeting, Anaheim 2004, Cell-063; Ribeiro et al., Natural Polymers and Composites IV, Proceedings from the International Symposium on Natural Polymers and Composites, Sao Pedro, 2002, pages 192 to 197; Kroger et al., Topics in Catalysis, 2000, 13(3), pages 237 to 242; Benvenuti et al., Applied Catalysis, A: General, 2000, 193 (1,2), pages 147 to 153; Grin et al., Khimicheskaya Fizika, 1994, 13(5), pages 113 to 118; Nakamura, Yoshio, Noguchi Kenkyusho Jiho, 1980, 23, pages 25 to 38; Musau et al., Biomass, 1987, Vol. 13, pages 67 to 74; Fayet et al., Carbohydrate Research, 1983, Vol. 122, pages 59 to 68; and FR 2 670 209 describe methods for preparation of hydroxymethylfurfural in the presence of a catalyst.

### Summary of the Invention

Provided herein is an improved method of preparing 2,5-(hydroxymethyl)furaldehyde comprising: i) combining a fructose source, a solvent selected from the group consisting of 1-methyl-2-pyrrolidinone, dimethylacetamide, dimethylformamide and combinations of thereof, with a catalyst to provide a reaction mixture; ii) heating said reaction mixture to a temperature and for a time sufficient to promote an acid-catalyzed dehydration reaction of fructose in said fructose source to form a product mixture; and iii) isolating 2,5-(hydroxymethyl)furaldehyde from said product mixture.

In another embodiment, there is provided a method of preparing 2,5-(hydroxymethyl)furaldehyde comprising: i) combining a fructose source, an organic solvent, and an acid catalyst to provide a reaction mixture; ii) heating said reaction mixture to a temperature and for a time sufficient to promote a dehydration reaction of fructose in said fructose source to form a first product mixture; iii) neutralizing the pH of the first product mixture to a pH of about 7 to 9; iv) distilling the first product mixture after neutralizing the pH to remove said organic solvent remaining in the first product mixture; and v) purifying said product mixture to provide a second product mixture comprising greater than 60% by weight of 2,5 - (hydroxymethyl)furaldehyde.

Also provided also herein is a method of preparing 2,5. (hydroxymethyl)furaldehyde comprising the steps of: i) combining a fructose source, an acid catalyst, a first organic solvent, and a second organic solvent that is non miscible with the first organic solvent to provide a reaction mixture, the first and second organic solvents being selected so that the second organic solvent preferentially dissolves 2,5-(hydroxymethyl)furaldehyde relative to the first organic solvent; ii) heating said reaction mixture to a temperature and for a time sufficient to promote a dehydration reaction of fructose in said fructose source to form a product mixture with a first immiscible phase and a second immiscible phase; and iii) isolating 2,5-(hydroxymethyl)furaldehyde from said second immiscible phase of said product mixture.

Provided herein is an improved method of preparing 2,5-(hydroxymethyl)furaldehyde. The method includes the steps of: i) combining materials comprising a fructose source, a solvent, and a catalyst to form a reaction mixture; ii) heating said reaction mixture to a temperature and for a time sufficient to promote an acid-catalyzed dehydration reaction of fructose in said fructose source to form a product mixture; and iii) isolating 2,5-(hydroxymethyl)furaldehyde from said product mixture. Preferably the catalyst is a heterogeneous, re-usable, or recyclable catalyst.

In one embodiment the fructose source is high fructose corn syrup, and the method is performed under vacuum conditions. In a further embodiment the carbohydrate source is added gradually in a stepwise fashion once the reaction has been initiated, this entails the addition of two or more discrete aliquots over a specified period of time. In an additional embodiment, the mixed carbohydrate source comprises a first carbohydrate source in a first physical state, and a second carbohydrate source in a second physical state, wherein the first and second physical states are not the same, that is to say they are in different physical states. Suitable carbohydrate sources include, but are not limited to, a hexose, a pentose, fructose syrup, crystalline fructose, and, process streams from the crystallization of fructose.

Suitable mixed carbohydrate source may comprise any industrially convenient carbohydrate sources, such as corn syrup. The mixed carbohydrate sources include, but are not limited to, hexoses, fructose syrup, crystalline fructose, high fructose corn syrup, crude fructose, purified fructose, high fructose corn syrup refinery intermediates and by-products, process streams from crystallizing fructose or glucose, and molasses, such as soy molasses resulting from production of soy protein concentrate.

Provided also herein is a further method of preparing 2,5-(hydroxymethyl)furaldehyde that includes the steps of: i) combining materials comprising a carbohydrate source, an organic solvent, and an ion-exchange resin catalyst to form a non-aqueous reaction mixture; ii) heating said non-aqueous reaction mixture to a temperature and for a time sufficient to promote a dehydration reaction of said carbohydrate source to form a first product mixture; iii) removing the ion-exchange resin catalyst from the first product mixture to provide a product isolate; iv) distilling the product isolate to remove said solvent remaining in said product isolate; and v) purifying said product isolate to provide a second product mixture comprising greater than 60% by weight of 2,5 - (hydroxymethyl)furaldehyde. In one embodiment, the product isolate is adjusted to a neutral pH after removing the ion-exchange resin from said product mixture, and before being subjected to a distillation to remove the organic solvent.

In one embodiment, the product mixture may be further isolated by such methods which are well known in the art, such as, but not limited to, filtration, vacuum or suction filtration, or gravity filtration. Purification of the product isolate may be carried out by a solvent extraction process to provide the second product mixture . Examples of solvent extraction processes that may be used include, but are not limited to, a column chromatography process and liquid-liquid extraction. A liquid-liquid extraction process comprises adding a mixture of a water-immiscible organic solvent and water to the product isolate to form an organic phase and an aqueous phase. This is followed by recovering the organic phase, and removing the water-immiscible solvent to yield purified 2,5-(hydroxymethyl) furaldehyde.

The possible extracting solvents include, but are not limited to, ethyl acetate, methyl isobutylketone, methyl ethyl ketone, methyl t-butyl ether, ethyl lactate, octanol, pentanol, and butyl acetate and combinations thereof. In a certain embodiment, the second product mixture comprises greater than 75% by weight of 2,5-(hydroxymethyl)furaldehyde. Yet another embodiment, the second product mixture comprises greater than 95% by weight of 2,5-(hydroxymethyl)furaldehyde.

In one embodiment, after product isolation the ion-exchange resin catalyst may be rinsed with the organic solvent used to carry out the reaction to recover product contained within the resin. After the rinse, the ion-exchange resin catalyst may be reused in a subsequent reaction. In a further embodiment, after product isolation the ion-exchange resin may be rinsed with a second organic solvent to recover product contained with in the resin. After the rinse, the ion-exchange resin catalyst may be reused in a subsequent reaction.

Provided also herein is a further method of preparing 2,5-(hydroxymethyl)furaldehyde. The method includes: i) combining materials comprising a carbohydrate source, a solvent and an ion-exchange resin catalyst to form a reaction mixture; ii) heating the reaction mixture to a temperature and for a time sufficient to promote a dehydration reaction of said carbohydrate source to form a first product mixture; iii) isolating the first product mixture to provide a product isolate. The method optionally comprises one or more of the following steps: iv) adjusting the product isolate to a neutral pH; v) adding a non-volatile flowing agent to the product isolate; vi) distilling the non-volatile flowing agent and the product isolate to remove the solvent from the product isolate; and vii) purifying the product isolate to provide a second product mixture comprising greater than 75% by weight of 2,5-(hydroxymethyl)furaldehyde.

In an embodiment, the purification of the product isolate may be performed by a process selected from the group consisting of short path distillation, thin film evaporation, wiped film evaporation, crystallization, and adsorption to an inert adsorbent. Adsorbents include, but are not limited to, silica, carbon, alumina, and other resins. A non-volatile flowing agent may be added to the product isolate to enhance separation. The non-volatile flowing agent may be chosen from the group consisting of polyethylene glycol, polyethylene glycol monoether, polyethylene glycol diether, and combinations thereof. In a further embodiment, the non-volatile flowing agent may be purified to a re-usable form after it has performed its role in the purification process. Such purification process may take place with the use of carbon as disclosed herein.

Provided also herein is a further method of preparing 2,5-(hydroxymethyl)furaldehyde. The method includes: i) combining materials comprising a carbohydrate source, a catalyst, a first organic solvent, and a second organic solvent to form a non-aqueous reaction mixture wherein said first organic solvent and said second organic solvent are immiscible in each other; ii) heating the non-aqueous reaction mixture to a temperature and for a time sufficient to promote a dehydration reaction of the carbohydrate source in said first organic solvent to form a product mixture with a first immiscible phase and a second immiscible phase; and iii) isolating 2,5-(hydroxymethyl)furaldehyde from said second immiscible phase of said product mixture.

In one embodiment of the above method, the second organic solvent is characterized by an ability to solubilize HMF in the presence of the first organic solvent, which is immiscible with regard to the second organic solvent and HMF. The second organic solvent may be selected from the group including, but not limited to, methyl isobutyl ketone, ethyl acetate, and chloroform. The first organic solvent is characterized as being less able to solubilize HMF than the second organic solvent when in contact with the second organic solvent; the result of which is a two-phase system. HMF is less soluble in said first immiscible organic phase than in said second immiscible organic phase. In one embodiment, the first organic solvent is dimethyl formamide.

The problem of the present invention is solved on the basis of claims 1 to 25.

### Detailed Description of the Invention

Reusable or recyclable catalysts are preferred for use in the reaction, as they provide for increased efficiency, and economic and industrial feasibility. As used herein, the term "recyclable catalyst" refers to a catalyst which is not irreversibly expended as a result of the reaction. In other words, the catalyst may be used again. Examples of recyclable or reusable catalysts include, but are not limited to, solid acid catalysts, ion-exchange resins, zeolites, Lewis acids, clays, and molecular sieves. Solid acid catalysts often comprise a solid material which has been functionalize to impart acid groups that are catalytically active. Solid acid catalysts may have a broad range of composition, porosity, density, type of acid groups and distribution of acid groups. Solid acid catalysts may be recovered and reused, optionally with a treatment to regenerate any activity that may have been lost in use. Some solid acid catalysts that may be used in the disclosed process include, but are not limited to Amberlyst 35, Amberlyst 36, Amberlyst 15, Amberlyst 131 (Rohm and Haas, Woodridge, IL), Lewatit S2328, Lewatit K2431, Lewatit S2568, Lewatit K2629 (Sybron Corp, Birmingham, NJ), Dianion SK104, Dianion PK228, Dianion RCP160, RCP21H, Relite RAD/F (Mitsubishi Chemical, White Plains, NY), and Dowex 50WX4 (Dow Chemical).

One example of a solvent that may be used is a polar solvent. The polar solvent maybe a polar aprotic solvent. Examples of possible solvents include, but are not limited to, 1-methyl-2-pyrrolidinone, dimethylacetamide, dimethylformamide, dimethyl sulfoxide, methyl ethyl ketone, methyl isobutylketone, acetonitrile, propionitrile, and combinations thereof.

In certain embodiments of the method, over 40% of hexoses present in the starting reactants are converted to HMF, the percent conversion being calculated by molar yield as described below. Yield may be increased by altering any of the variables, such as solvent type, concentration, catalyst, time and/or temperature of the reaction conditions, etc. It has been further found that the gradual removal of water from the dehydration reaction increases the yield of HMF. The dehydration of fructose to HMF occurs with the loss of three water molecules, and the formation of three points of non-saturation, or double bonds (two alkene bonds, and the carbonyl group). By removing water as it is formed, side-reactions are thereby minimized, and an increased yield has been observed. Water removal may take place via evaporation. A rotary evaporation machine may be employed to promote water removal. The use of a rotary evaporator, or "rotovap," is well-known in the art. Water removal may also be carried out by evaporation from the reaction mixture and condensation as ice or water on a cold finger or reflux condenser. Water may also be removed by distillation, including azeotropic distillation with a water-entraining solvent which may optionally be stripped of water and the water depleted solvent returned to the reaction vessel. A suitable distillation apparatus, such as a Barrett type receiver may also be employed. A water-absorbing material may also be used to remove water. Such materials are well-known in the art, and include, but are not limited to, molecular sieves.

In one embodiment, the reactions disclosed herein are performed at moderately high temperatures, typically in a range of from about 95° to about 125°C. In a further embodiment, the temperature range is from about 105° C to about 115° C. It is preferable to use temperatures below 200 degrees Celsius. The reactions disclosed herein typically occur in a time frame of from about one to about six hours. More typically, the reactions take from about two hours to about five and a half hours. If additional steps regarding the isolation and purification of HMF are preformed, additional time may be required.

As used herein, the term "zeolite" refers to a hydrated silicate of aluminum and one or both of sodium and calcium. Examples include, but are not limited to, analcite, chabazite, heulandite, natrolite, stilbite, thomsonite, in either powder or pellet form. Commercial zeolites products include, but are not limited to, CBV 3024 and CBV 5534G (Zeolyst International), T-2665, T-4480 (United Catalysis, Inc), LZY 64 (Union Carbide), and H-ZSM-5 (PQ Corporation).

As used herein, Cornsweet 90 refers to a high fructose corn syrup product of commerce nominally containing 60% to 70% fructose. High fructose corn syrup refinery intermediate and by-product is a fructose-rich stream generated in a fractionation system positioned after an isomerization column in the production of high fructose corn syrup. A suitable process stream from crystallizing fructose is called "mother liquor" and comprises a solution of fructose in ethanol. Typically this process stream is about 24% solids, almost all of the solids being fructose, and contains about 60% ethanol. For use in HMF production, the ethanol can be removed from the mother liquor. A similar mother liquor from glucose crystallization contains about 50% solids. Mixed carbohydrate sources can be obtained by blending carbohydrates, such as by adding crystalline fructose to high fructose corn syrup.

As used herein, "reaction yield" is calculated using the equation (moles of product/moles of starting material)*100. Product purity is reported on a weight percent basis.

As used in this equation, "starting material" refers to the fructose present in the carbohydrate source, mixed carbohydrate source, or other reactant for the particular dehydration reaction.

As used herein, the term "fructose source" refers to a material that comprises sucrose. Typical embodiments are solutions having at least 25% sucrose by solute weight, and which may include other materials such as other carbohydrate compounds. Preferably, the carbohydrate compounds are hexoses. The versatility of the reaction conditions provided herein allow an industrially convenient source to be used as the starting material, that is to say, the reaction is not limited to a particular carbohydrate source or to fructose of high purity.

Suitable fructose sources typically include high fructose corn syrup (HFCS) or any HFCS refining process stream that includes at least 25% sucrose. HFCS is typically commercially available in products comprising solutions having 42% to 95% fructose by solute weight which are typically sold for use as industrial scale sweeteners. The most economical embodiments of the invention use HFCS having about 90% sucrose by solute weight. However, less economical embodiment's invention can be practiced with sources having less sucrose by weight. To improve economic efficiencies, less pure sucrose sources can be conveniently blended with higher purity sucrose sources or even crystalline sucrose to achieve a solution having at least 25% sucrose by solute weight.

Optional neutralization of the product isolate is carried out by addition of a suitable alkali substance, such as a basic ion exchange resin, potassium hydroxide, or sodium hydroxide. This neutralization step allows for subsequent product recovery by distillation without heat-catalyzed degradation or polymerization, resulting in the elimination of tarry degradation products and resinous solids being formed in distillation. This neutralization step also allows for subsequent product recovery with a flowing agent without heat-catalyzed degradation or polymerization, resulting in the elimination of tarry degradation products and resinous solids being formed in distillation.

HMF can be purified from reaction mixtures by removal of catalyst resin and forming a product isolate, neutralizing the product isolate, removing solvent from product isolate by distillation, and treating the resulting distillant with water and an organic solvent. HMF partitions to the organic solvent and can be recovered with purity in excess of 95% by weight. This level of purity has not been obtained by other processes.

After HMF has been purified from reaction mixtures by removal of the solid acid catalyst and forming a product isolate, the solid acid catalyst may be rinsed with the organic solvent used to carry out the reaction to recover product contained within the catalyst. After the rinse, the solid acid catalyst may be reused in a subsequent reaction. In a preferred embodiment, after HMF has been purified from reaction mixtures by removal of the solid acid catalyst forming a product isolate, the solid acid catalyst may be rinsed with a second organic solvent to recover product contained with in the catalyst. After the rinse, the solid acid catalyst may be reused in a subsequent reaction.

Purity was determined by ¹³C NMR and Proton NMR, in some cases by capillary GC, and in some cases by UV adsorption.

As used herein, the term "non-aqueous mixture" refers to a mixture comprising a non-aqueous solvent and at least one other component, wherein the content of the solvent is greater than the content of the at least one other component, as measured by volume. The at least one other component may comprise, a water-containing substrate, such as HFCS, or an organic solvent. Non-aqueous solvents are usually measured by volume, and other components are usually measured by weight.

As used herein, the term "isolate" refers to the process of preservation of a material originally present in a product mixture after the product mixture has been subjected to a step to remove other material from the product mixture, as well as the isolated material resulting from the process. Examples of "other material" that is removed includes solid material, such as catalyst by methods including the processes of filtration, decantation, centrifugation, and washing. Filtration may be performed by one of the processes selected from the group comprising but not limited to gravity filtration, vacuum filtration, and suction filtration.

The term "non-volatile flowing agent" as used herein refers to an inert material which, when added to a product mixture, aids in the recovery of the desired compound by distillation. In certain embodiments, the fugacity of the flowing agent is sufficiently low so that it will not volatilize as the target product is removed by evaporation.

The formation of two immiscible solvent phases in the reaction mixture facilitates purification of an HMF product. Solvents can be easily classified on the basis of polarity. One such measure of polarity is the Log P value. Log P is defined as the partition coefficient of a given compound in a two-phase system of water and octanol. Log P can be determined experimentally or calculated from hydrophobic fragmental constants according to standard procedures (Hansch, C. & Leo, A (1979) Substituent constants for correlation analysis in chemistry and biology. John Wiley & Sons, New York NY; Leo, A., Hansch, C. & Elkins, D. (1971) Chem. Rev. 71, 525;Rekker, R. F. (1977) The hydrophobic fragmental constant, Elsevier, Amsterdam; Rekker, R. F. & de Kort, H. M. (1979) Eur. J. Med. Chim. 14, 479).

Preferred two-phase organic solvent systems include a first solvent having a log P value of less then zero and a second solvent having a log P value in the range of about 0.4 to about 3.4; a further two-phase organic solvent systems include a first solvent having a log P value in the range of about -0.75 to about -1.95. In a further embodiment the second solvent has a log P value in the range of from about 0.6 to about 2.7, and in an additional embodiment, the two-phase organic solvent systems include a first solvent having a log P value of about -1.04 and a second solvent having a log P value of about 1.32. A suitable two-phase organic solvent system comprises a first phase of dimethylformamide and a second phase of methyl isobutyl ketone. Table 1 provides Log P data for certain solvents.

Methyl isobutyl ketone is generally miscible with a broad range of solvents (J. S. Drury (1952) Miscibility of solvent pairs, Industrial and Engineering Chemistry 44:11, page C-684). Solvents immiscible with methyl isobuyl ketone include diethanolamine, ethylene glycol, glycerol and trimethylene glycol. None of these solvents are suitable for the intended reaction because of their reactivity.

**Table 1. Log P data for some solvents**

| **Solvent** | **Log P *** |
|---|---|
| 1, 2-Dichlorobenzene | 3.38 |
| Carbon tetrachloride | 2.83 |
| Toluene | 2.69 |
| Chloroform | 2.24 |
| Benzene | 2.03 |
| 2-Heptanone | 1.83 |
| Butyl acetate | 1.71 |
| 1,2-Dichloroethane | 1.48 |
| Methyl isobutyl ketone | 1.32 |
| Dichloromethane | 1.25 |
| Ethyl propionate | 1.21 |
| 2-Pentanone | 0.91 |
| Diethyl ether | 0.89 |
| t-Amyl alcohol | 0.89 |
| Butanol | 0.88 |
| Cyclohexanone | 0.81 |
| Ethyl acetate | 0.66 |
| Pyridine | 0.64 |
| Tetrahydrofuran | 0.46 |
| 2-Butanone | 0.29 |
| 2-Propanol | 0.05 |
| Acetone | -0.24 |
| Dioxane | -0.27 |
| Ethanol | -0.32 |
| Acetonitrile | -0.34 |
| Methanol | -0.77 |
| N, N-Dimethylformamide | -1.04 |
| Dimethyl sulfoxide | -1.35 |
| Formamide | -1.51 |
| Ethylene glycol | -1.93 |

| | |
|---|---|
| * Log P values were taken from Hansch, C. & Leo, A (1979) Substituent constants for correlation analysis in chemistry and biology. John Wiley & Sons, New York NY; Leo, A., Hansch, C. & Elkins, D. (1971) Chem. Rev. 71, 525. | |

As used herein, the term "crude reaction mixture" refers to an unrefined or unpurified composition.

### Examples

The following are examples of the dehydration of a fructose source to a furan derivative or organic acid, as well as isolation and/or purification techniques to optimize product recovery of increased product yield.

### EXAMPLE 1

### PREPARATION OF HMF FROM HIGH FRUCTOSE CORN SYRUP AT 115°C in N-Methylpyrrolidinone (NMP)

A 250 mL 3-neck round bottom flask was fitted with a magnetic stir bar, heating mantle, reflux condenser, and temperature probe. To this flask was charged 100 mL of NMP (Aldrich) and 20 g of Amberlyst 35 resin (Rohm and Haas, Woodridge, IL). Amberlyst 35 is a macroreticular, strongly acidic, polymeric catalyst. The mixture was heated to 115°C, and 50 g of Cornsweet 90 (HFCS, ADM, Clinton IA) was added. Heating continued in this manner at 115°C over a 5 hour period. Water condensed on the reflux condenser. After 5 hours, the contents of the flask were cooled to about 70°C, and the resin removed by vacuum filtration to provide a product isolate. The product isolate was analyzed to provide a solution of 14.2% HMF by weight and 4.7% fructose. Calculations indicate an 80.6% molar yield of HMF from fructose and 94.1 % conversion.

### EXAMPLE 2

### PREPARATION OF HMF FROM HIGH FRUCTOSE CORN SYRUP AT 105°C in NMP

This example illustrates the effect of temperature on the dehydration of fructose to HMF. A 250 mL 3-neck round bottom flask was fitted with a magnetic stir bar, heating mantle, reflux condenser, and temperature probe. To this flask was charged 100 mL of NMP (Aldrich) and 20 g of Amberlyst 35 resin (Rohm and Haas, Woodridge, IL). The mixture was allowed to heat to 105°C, and 50 g of Cornsweet 90 (HFCS, ADM, Clinton, IA) was added. Heating continued in this manner at 105°C over a 5 hour period. Water condensed on the reflux condenser. After 5 hours, the contents of the flask were cooled to about 70°C, and the resin removed by vacuum filtration to provide a product isolate. The product isolate was analyzed to provide a solution of 12.9% HMF and 3.9% fructose. Calculations indicate a 71.6% molar yield of HMF from fructose and 85.4% conversion.

### EXAMPLE 3

### PREPARATION OF HMF FROM HIGH FRUCTOSE CORN SYRUP AT 105°C in NMP UNDER VACUUM CONDITIONS

This example illustrates the effect of distillation on the dehydration of fructose to HMF. A 250 mL 3-neck round bottom flask was fitted with a magnetic stir bar, heating mantle, condenser, temperature probe, and receiving flask. To this flask was charged 100 mL of NMP (Aldrich), 20 g of Amberlyst 35 resin (Rohm and Haas, Woodridge, IL), and 50 g of Cornsweet 90 syrup. The mixture was heated to 105°C under house vacuum. The distillate was collected. After 2 hours, the contents of the flask were cooled to about 80°C, and the resin removed by vacuum filtration to provide a product isolate. The product isolate was analyzed to provide a solution of 14.2% HMF and 1.1% fructose. Calculations indicate a 75.7% molar yield of HMF from fructose and 79.5% conversion.

### EXAMPLE 4

### PREPARATION OF HMF FROM HIGH FRUCTOSE CORN SYRUP AT 115°C IN NMP

This example illustrates the effect of distillation on the dehydration of fructose to HMF. A 2L 3-neck round bottom flask was fitted with a magnetic stir bar, heating mantle, condenser, temperature probe, and receiving flask. To this flask was added 500 mL of NMP (Aldrich), 200 g of Amberlyst 35 wet resin (Rohm and Haas, Woodridge, IL), and 500 g of Cornsweet 90. The mixture was heated to 115°C and subjected to vacuum distillation under house vacuum. After 4 hours, the resin was removed by filtration to provide a product isolate of 729.68 g of 20.4% HMF. Calculations indicate a 68.6% yield of HMF.

### EXAMPLE 5

### PREPARATION OF HMF FROM HIGH FRUCTOSE CORN SYRUP AT 105°C in DMAc

This example illustrates the effect of solvent on the dehydration of fructose to HMF. A 250 mL 3-neck round bottom flask was fitted with a magnetic stir bar, heating mantle, reflux condenser, and temperature probe. To this flask was charged 100 mL of DMAc (Aldrich) and 20 g of Amberlyst 35 resin (Rohm and Haas, Woodridge, IL). The mixture was heated to 105°C, and 50 g of Cornsweet 90 (HFCS, ADM, Clinton, IA) was added. Heating was continued in this manner at 105°C over a 5 hour period. Water was condensed on the reflux condenser. After 5 hours, the contents of the flask were cooled to about 90°C, and the resin was removed by vacuum filtration to provide a product isolate. The product isolate was analyzed to provide a solution of 13.5% HMF and 6.0% fructose. Calculations indicate 62.1% molar yield of HMF from fructose and 74.6% conversion.

### Reference EXAMPLE 6

### PREPARATION OF EMF FROM FRUCTOSE IN BATCH MODE

A 500 mL round bottom flask equipped with a reflux condenser, temperature probe, and magnetic stir bar was charged with a solution of 30 g fructose (Aldrich), 225 mL HPLC grade ethanol (Aldrich), and 30 g of Amberlyst 131 resin (Rohm and Haas). Amberlyst 131 is a strongly acidic polymeric catalyst with a particle size of 0.7-0.8 mm and water content of 65%. The stirred mixture was heated to reflux for 24 hours. At this time, the slurry was filtered and the resin washed with ethanol to provide 174 mL of product isolate containing 5.4 g/L HMF and 61.6 g/L EMF.

### Reference EXAMPLE 7

### PREPARATION OF EMF FROM FRUCTOSE VIA COLUMN ELUTION

A 100 mL glass liquid-chromatography column (2.54 cm I.D) was slurry packed in HPLC grade ethanol (EtOH) with Amberlyst 131 resin obtained from Rohm and Haas Company (Woodridge, IL). The resin was washed with 500 mL of EtOH. The final packed volume was 100 ml. The feed material consisted of 5 mL of a 20% solution of fructose in EtOH. The feed was then loaded on the resin column by gravity flow and fractions were eluted. The column was maintained at 60°C and elution at 0.6 mL/min. Table 2 summarizes the results of this study. A complete conversion of fructose to a mixture of HMF/EMF was achieved, with the major product being EMF.

**Table 2. Column Synthesis of EMF from Fructose using Amberlyst 131 Resin.¹**

| **Fraction #** | **Volume (mL)** | **Fructose (ppm)** | **HMF (ppm)** | **EMF (ppm)** |
|---|---|---|---|---|
| 2 | 8 | 0 | 0 | 0 |
| 5 | 13.6 | 0 | 0 | 0 |
| 7 | 21.6 | 0 | 0 | 294 |
| 9 | 32.1 | 262 | 370 | 1,862 |
| 11 | 40.1 | 79 | 420 | 2,613 |
| 13 | 48.1 | 134 | 364 | 4,451 |
| 15 | 57.1 | 119 | 794 | 6,008 |
| 17 | 65.6 | 120 | 615 | 6,385 |
| 19 | 73.6 | 0 | 308 | 4,293 |
| 21 | 82.1 | 0 | 0 | 1,488 |
| 24 | 94.1 | 0 | 0 | 276 |
| 26 | 102.1 | 0 | 0 | 60 |

| | | | | |
|---|---|---|---|---|
| ¹Column was maintained at 60° C with a steady flow rate of 0.6 mL/min. | | | | |

### Reference EXAMPLE 8

### PREPARATION OF EMF FROM FRUCTOSE VIA COLUMN ELUTION

This example illustrates the effect of change in resin to Amberlyst 35 obtained from Rohm and Haas Company (Woodridge, IL). Amberlyst 35 is a macroreticular, strongly acidic, polymeric catalyst. The feed material was prepared and loaded on to the column by gravity flow as described in Example 7. The column was maintained at 60°C and the elution was carried out at 0.6 mL/min. A summary of this is provided in table 3. Nearly 85% of the starting fructose was converted into a mixture of HMF/EMF with the major product being EMF.

**Table 3. Column Synthesis of EMF from Fructose using Amberlyst 35 Resin.¹**

| **Fraction #** | **Volume (mL)** | **Fructose (ppm)** | **Ethyl Levulinate (ppm)** | **HMF (ppm)** | **EMF (ppm)** |
|---|---|---|---|---|---|
| 1 | 2.0 | 263 | 242 | 263 | 263 |
| 3 | 13.0 | 271 | 249 | 271 | 271 |
| 5 | 25.0 | 230 | 212 | 230 | 230 |
| 7 | 34.5 | 253 | 233 | 253 | 253 |
| 8 | 37.5 | 227 | 209 | 227 | 579 |
| 10 | 46.5 | 2,737 | 948 | 1,180 | 5,687 |
| 12 | 58.0 | 2,507 | 203 | 1,157 | 7,844 |
| 14 | 67.0 | 1,970 | 1,526 | 1,186 | 9,526 |
| 16 | 76.0 | 520 | 246 | 325 | 2,023 |
| 18 | 85.0 | 282 | 260 | 282 | 282 |
| 19 | 89.5 | 256 | 236 | 256 | 256 |
| 20 | 96.5 | 269 | 248 | 269 | 269 |

| | | | | | |
|---|---|---|---|---|---|
| ¹Column was maintained at 60° C with a steady flow rate of 0.6 mL/min. | | | | | |

### Reference EXAMPLE 9

### PROCESS FOR THE SYNTHESIS AND PURIFICATION OF EMF

Dehydration: Amberlyst 131 Wet (145 g) was dried in vacuum at 85°C for three days. This catalyst was combined with 117 g crystalline fructose and 468 g of 100% ethanol in a steel reactor. With stirring at 600 rpm, the reaction mixture was gradually heated to 110°C over 30 minutes. The temperature was maintained for 45 minutes, and then the reaction mixture was cooled to ambient temperature over 7 minutes. The catalyst was filtered from the red-black reaction mixture, and the reaction mixture was treated with a rotary evaporator under house vacuum to remove ethanol.

Distillation of EMF on Wiped-Film Evaporator: Poly(ethylene glycol)-400 (47 g) was added to the dark residue (89 g). EMF was distilled from this mixture on a wiped-film evaporator at 110°C, 4.7 mm Hg, and 400 rpm, yielding a yellow distillate (68 g) containing EMF (44 g, 44% molar yield from fructose), ethyl levulinate (20 g, ELA), and ethanol (5 g). NMR (δ, 1 H): 9.54, (s, 0.8 H) EMF; 7.16, (d, 1.0 H), EMF; 6.46, (s, 1.0 H), EMF; 4.46, (s, 2.0 H), EMF; 4.05, (quartet, 1.0 H) ELA; 3.63, (quartet, 0.7 H), EtOH; 3.52, (quartet, 2.0 H), EMF; 2.68, (t, 1.1 H), ELA; 2.49, (t, 1.2 H), ELA; 2.12, (s, 1.6 H), ELA; 1.17, (m, 5.6 H), ELA, EMF, EtOH.

### EXAMPLE 10

### PREPARATION OF HMF FROM FRUCTOSE USING A TWO-PHASE ORGANIC SOLVENT SYSTEM

A 500 mL round bottom three neck flask was equipped with a reflux-condenser, temperature probe, and a magnetic stir bar. To this flask was added 5 g of fructose, 5 g of Amberlyst 35 resin, and a first organic solvent comprising 50 mL of dimethylformamide (DMF) and a second organic solvent comprising 200 mL of methyl isobutyl ketone (MIBK). The reaction was heated to 85°C for 7h. The mixture was cooled and filtered. The resin was washed with small quantities of MIBK. The two layers were separated and the product isolate (155 mL) in the MIBK phase contained 17.9 g/L HMF to provide an overall yield of 89.3%.

### Reference EXAMPLE 11

### REPARATION OF LEVULINIC ACID FROM HIGH FRUCTOSE CORN SYRUP USING ACIDIC RESIN CATALYSTS

A 250 mL round bottom three neck flask was equipped with a reflux-condenser, temperature probe, and a magnetic stir bar. To this flask was added 50 g of Cornsweet 90 syrup, 20 g of Amberlyst 35 resin, and 100 mL of poly(ethyleneglycol) dimethyl ether-500. The mixture was heated to 100°C for 4h. The mixture was cooled and filtered to provide an overall yield of 45.3% levulinic acid.

### Reference EXAMPLE 12

### PREPARATION OF LEVULINIC ACID FROM FRUCTOSE USING ACIDIC RESIN CATALYSTS

A solution of crystalline fructose (30 g, 90%) in water (500 mL) was placed in a 1L autoclave reactor. To this reactor was added 60 g of Amberlyst 35 Wet resin. The solution was stirred (500 rpm) and heated to 150°C. After 4.5 hours, the reactor was cooled and the solution was filtered to remove the catalyst to provide a product isolate. The dark brown product isolate (72.04 g) contained 149.83 g/kg levulinic acid to provide an overall yield of 62% levulinic acid from fructose.

### Reference EXAMPLE 13

### PREPARATION OF LEVULINIC ACID FROM HIGH FRUCTOSE CORN SYRUP USING ACIDIC RESIN CATALYSTS

A solution of Cornsweet 90 (45.24 g) in water (500 mL) was placed in a 1L autoclave reactor. Amberlyst 35 Wet resin (60 g) was added and the mixture stirred (500 rpm). After 18 hours, the reactor was cooled and the solution filtered to provide a product isolate. The product isolate was treated with a rotary evaporation machine to remove the solvent, and provided 17.98 g of dark brown oil containing 467.22 g/kg levulinic acid for a yield of 41.2%.

### EXAMPLE 14

### PROCESS FOR THE PREPARATION AND PURIFICATION OF HMF FROM HFCS

Step 14a. Neutralization: A 202.7 g sample of product isolate prepared as described in Example 4 was placed in a 500 mL Erlenmeyer flask, and 25.0 g of poly(ethylene glycol)-600 was added to serve as a flowing agent in later purification. The mixture was stirred continuously for 30 minutes at ambient temperature and neutralized with the gradual addition of Amberlyst A26OH resin (Rohm and Haas) before being subjected to distillation to remove solvent. Amberlyst A26OH is a strong base, type 1, anionic, macroreticular polymeric resin. The pH of the crude product mixture was increased to 7.5-8.0 by the application of Amberlyst A26OH resin. The Amberlyst A260H resin was then removed by filtration.

Step 14b. Distillation of DMAc: The solvent (DMAc) was distilled from the neutralized product mixture under vacuum (4-6 torr) at 100°C using a 4" Vigreaux column with six tiers. A brown residue containing HMF and poly(ethylene) glycol (64.5 g, 28.8% HMF) remained in the distillation vessel and 150 g of distilled DMAc were isolated.

Step 14c. Short Path Distillation of HMF: The brown residue containing HMF (145.5 g, 28.8% HMF) obtained from fractional distillation in step 14b was subjected to short path distillation at 150°C and 0.014-0.021 torr. A yellow distillate (96% purity-HMF, 47.4 g) and brown residue (79.6 g) were isolated. The distillate crystallized upon cooling. NMR (δ, 1 H): 9.49, (s, 1 H); 7.16, (d, 1.0 H); 6.46, (s, 1.0 H); 4.62, (s, 2.0 H).

Regeneration of PEG for re-use: A 12.5 g portion of the dark brown PEG residue obtained from the short path distillation was treated with 50 mL of hot water and 24 g of carbon (Calgon, CPG-LF 12X40). NMR indicated that the dark brown PEG residue was composed of greater than 95% PEG. The mixture was allowed to stir for three days. The mixture was vacuum filtered to remove the carbon and 8.4 g of a clear yellow oil resembling the starting PEG in appearance was isolated. NMR indicated that the purity of the recovered PEG was 100%.

### EXAMPLE 14a

### PROCESS FOR THE PURIFICATION OF HMF FROM HFCS

A 180 g sample of material prepared and neutralized as described in Example 4 was added to 20 g poly(ethylene glycol) dimethyl ether-500 flowing agent. This material was subjected to short path distillation at 150°C and 5 mbar. A yellow distillate (67.4% purity HMF, 11.92 g) and brown residue (191 g) were isolated.

### EXAMPLE 15

### PROCESS FOR THE PURIFICATION OF HMF FROM HFCS

Step 15a. Neutralization: An HMF product isolate as prepared in example 4 was neutralized with the gradual addition of aqueous sodium hydroxide (pH 7.5) before being subjected to distillation to remove solvent.

Step 15b. Fractional Distillation to remove NMP: The neutralized product isolate was subjected to distillation under reduced pressure (4-6 torr) at 115°C using a 4" Vigreaux column with six tiers to remove solvent (NMP). A purified product isolate comprising a brown residue (264.6 g) and 490 g of distilled NMP were obtained.

Step 15c. Solvent Extraction: A 30.25 g sample of purified product isolate (brown residue prepared in step 15b), 45 mL of ethyl acetate, and 15 mL of water were placed in a 125 mL Erlenmeyer flask and allowed to stir at ambient temperature. After 20 min, the mixture was transferred to a separatory funnel and the two layers separated. The ethyl acetate layer was removed and the aqueous layer was washed with 20 mL of ethyl acetate, the organic layers were combined and dried over MgSO₄. The dried combined organic layer was filtered and the solvent evaporated to provide 15.91 g of bright red oil which was 84.2% purity HMF.

### EXAMPLE 16

### PROCESS FOR THE PURIFICATION OF HMF FROM HFCS

Solvent Extraction: A 37.4 g sample of the solvent stripped material prepared as described in Example 15, 47 mL of methyl isobutylketone (MIBK), and 9.6 mL of water were placed in a 125 mL Erlenmeyer flask and allowed to stir at ambient temperature. After 20 min, the solution was transferred to a separatory funnel and the two layers separated. The aqueous phase was washed with 20 mL of MIBK and the organic phases combined and dried over MgSO₄. The solution was filtered and the solvent evaporated to provide 24.33 g of bright red oil which was 88% purity HMF in 97% yield.

### EXAMPLE 17

### PROCESS FOR THE PREPARATION AND PURIFICATION OF HMF FROM FRUCTOSE

Step 17a. Dehydration: Amberlyst 35 Dry (20 g) was combined with 40 g crystalline fructose and 200 mL of acetonitrile (ACN) in a three neck flask equipped with a reflux condenser, temperature probe, and magnetic stir bar. The reaction mixture was heated to reflux (80° C). The temperature was maintained for 5 hours, and then the reaction mixture was cooled. The catalyst was filtered and washed with acetonitrile to provide a product isolate. The product isolate was subjected to rotary evaporation to provide for evaporation of the solvent and 17.6 g of brown oil containing 33.9% HMF.

### Step 17b. Chromatographic Purification of HMF from ACN Reaction

Mixture: A glass-liquid chromatography column (2.54 cm I.D) was packed in heptane with C-Gel 560, 60-200 µ silica (Uetikon, Switzerland). The feed material for chromatographic separation using silica gel was prepared by dissolving the dehydration product (2.95 g) in 10 mL of 80:20 heptane:acetone solution. The feed material was loaded on the silica column by gravity flow and fractions were eluted including those shown in Table 3.

**Table 4. Chromatographic Purification of HMF from Crude ACN Reaction Mixture.¹**

| **Fraction #** | **Fructose** | **Fructose (ppm)** | **HMF (ppm)** | **Formic Acid (g/kg)** | **Levulinic Acid (g/kg)** |
|---|---|---|---|---|---|
| 1 | 10 | 967 | 23,239 | 0.70 | 0.00 |
| 5 | 4 | 384 | 476,872 | 1.53 | 5.38 |
| 7 | 0 | 0 | 826,108 | 0.00 | 0.00 |
| 8 | 0 | 0 | 814,378 | 0.00 | 11.59 |
| 9 | 0 | 0 | 622,706 | 0.00 | 17.62 |
| 10 | 22 | 2215 | 101,450 | 0.00 | 43.26 |
| 11 | 14 | 1386 | 70,241 | 0.00 | 15.22 |
| 12 | 13 | 1264 | 90,195 | 0.00 | 11.93 |
| 13 | 23 | 2252 | 40,207 | 4.03 | 12.14 |
| 14 | 28 | 2782 | 20,817 | 7.30 | 8.84 |
| 15 | 25 | 2521 | 30,723 | 9.20 | 1.05 |
| 16 | 35 | 3457 | 23,024 | 0.00 | 0.00 |
| 17 | 58 | 5847 | 26,892 | 20.66 | 0.00 |
| 18 | 88 | 8799 | 38,819 | 12.46 | 0.00 |
| 19 | 128 | 12837 | 32,050 | 8.66 | 0.00 |
| 20 | 116 | 11590 | 33,163 | 22.43 | 0.00 |

| | | | | | |
|---|---|---|---|---|---|
| ¹Samples were eluted from C-Gel 560, 60-200 microns using heptane:acetone gradient system. | | | | | |

Hence, by gradient elution of the column, isolated fractions with HMF content of >81% were obtained.

### EXAMPLE 18

### PROCESS FOR THE PURIFICATION OF HMF

A 2.0 g sample of HMF (21 %) was prepared in as in the dehydration Step 17a. of Example 17 using HFCS, treated with MIBK (2 mL) and water (1 mL), and the layers were separated. The organic layer was dried over MgSO₄, filtered, and the solvent evaporated to provide a bright red extract of 78.9% HMF purity with 93.9% recovery of HMF from the crude material.

### EXAMPLE 19

### PREPARATION OF HMF FROM HFCS USING ACIDIC RESIN CATALYSTS AT 115°C

This example illustrates the effect of resin on the dehydration of fructose to HMF. A summary of data is shown in Table 5. A 250 mL 3-neck round bottom flask was fitted with a magnetic stir bar, heating mantle, reflux condenser, and temperature probe. To this flask was charged 50 mL of NMP (Aldrich), 50 g of polyethylene glycol dimethyl ether-600, and 50 g of HFCS. The mixture was allowed to heat to 65°C, and 20 g of Dianion RCP160M resin (Mitsubishi Chemical America, Inc.) was added. Dianion RCP160M resin is a strongly acidic polymeric catalyst with a particle size distribution of 250 - 710 µm and a water retention of 45-55%. Heating continued in this manner at 115°C over a 4 hour period. Water condensed on the reflux condenser. After 4 hours, the contents of the flask were cooled to about 90°C, and the resin removed by vacuum filtration to provide a product isolate. The product isolate was analyzed and found to be a solution of 17.5% HMF and 0.2% fructose. Calculations indicate 74.5% molar yield of HMF from fructose and 77.1 % conversion.

**Table 5. Comparison of HMF Conversion with Various Resins.¹**

| **Reference #** | **Time (h)** | **Resin** | **HMF Yield (%)** | **Conversion (%)** |
|---|---|---|---|---|
| 4474-58 | 1 | RAD/F | 54.1 | 60.1 |
| | 2 | RAD/F | 63.5 | 67.3 |
| 4474-59 | 1 | RCP160M | 61.2 | 63.9 |
| | 2 | RCP160M | 74.5 | 77.1 |
| 4474-29 | 1 | Amberlyst 35 | 34.6 | 46.9 |
| | 2 | Amberlyst 35 | 57.1 | 70.2 |

| | | | | |
|---|---|---|---|---|
| ¹Reactions were performed with HFCS, in NMP/PEGE at 115° C. | | | | |

### EXAMPLE 20

### PROCESS FOR THE PREPARATION AND PURIFICATION OF HMF FROM HFCS

A 10 g sample of solvent stripped material as prepared in Example 15, step 15b (42% HMF purity) was placed in 50 mL of distilled water and 10 g of an inert adsorbent (Calgon CPG 12X40 carbon) was placed in a beaker and allowed to stir at room temperature for 12 hours. HMF adsorbs light at 284 nm. UV analysis (λ = 284 nm) after 12 hours of stirring indicated HMF had been adsorbed from the mixture. The carbon was collected by filtration, washed with water, and then allowed to stir at room temperature in 50 mL of acetone to desorb HMF. After 12 hours, the carbon was removed by filtration and the filtrate evaporated to provide 3.31 g of material with 80.1% HMF purity.

### EXAMPLE 21

### PROCESS FOR THE PREPARATION AND PURIFICATION OF HMF FROM HFCS

A 33.0 g sample of solvent stripped material as prepared in Example 15 (42% HMF purity) was treated with 35 g of an inert adsorbent (Calgon CPG 12X40 carbon) in 165 mL of distilled water. The mixture was allowed to stir at room temperature for 12 hours. The carbon was removed by Buchner filtration, rinsed with water, and dried under vacuum. The dried carbon was subjected to Soxhlet extraction using 600 mL of acetone for 18 hours to desorb HMF. The solvent was evaporated to provide 18.41 g of deep red oil having an HMF purity of 67.1%. The total recovery of HMF was 90.1%.

### EXAMPLE 22

### PREPARATION OF HMF FROM HFCS USING ROTARY EVAPORATION

This example illustrates the effect of rotary evaporation on the dehydration of fructose to HMF. To a 500 mL round bottom flask was charged 100 mL of NMP (Aldrich), 25 g of high-fructose corn syrup, and 15 g of wet Amberlyst 35 resin. An oil bath was heated to 120°C, and the flask rotated in the bath, under vacuum of 200 mm Hg. Rotary evaporation continued in this manner at 120°C over a 1 hour period. Distillate was collected. After 1 hour, the contents of the flask were subjected to Buchner filtration to remove the resin to provide a product isolate. The product isolate was analyzed to show 7.1% HMF, 91.5% NMP, and 1.9% water. Calculations indicate 88.6% molar yield of HMF from HFCS and 90.7% conversion.

### EXAMPLE 23

### PREPARATION OF HMF FROM HFCS WITH MOLECULAR SIEVES

This example illustrates the usefulness of molecular sieves as drying agents in the production of HMF from HFCS. To a 3-neck 500 round bottom flask equipped with a condenser, temperature probe, and stirring bar, was added 100 mL of NMP, 50 g of HFCS, 20 g of wet Amberlyst 35 resin, and 20 g of UOP 3A molecular sieves. The reaction was heated to 105°C and let stir under these conditions for 1 hour. Results indicate a 10.6% HMF solution providing an overall yield of 60.6%. The addition of sieves to promote the removal of water during the reaction allows for a faster conversion of HFCS to HMF.

### EXAMPLE 24

### PREPARATION OF HMF WITH GRADUAL ADDITION OF HFCS TO REACTION MIXTURE

This example illustrates the effect of gradual addition of HFCS to a heated reaction mixture. A 3-neck 500 mL round bottom flask was fitted with a dropping funnel, temperature probe, and a jacketed condenser with distilling head. To this flask was added 100 mL of NMP and 40 g of wet Amberlyst 35 resin. The flask was heated to 130°C with vacuum and the feed material (100 g of HFCS in 50 mL of NMP) was added dropwise over 1.5 hours. Upon complete addition of the feed, the reaction continued with vigorous stirring for 3 hours. At this time, the reaction was cooled to 90°C, and the resin removed via Buchner filtration (#415 VWR paper) to provide a product isolate. Results indicate a product isolate of 10.4% HMF, 85.4% NMP, and 2.76% water. Thus, a 77.8% molar yield of HMF was obtained.

### Reference EXAMPLE 25

### PREPARATION OF 2,5-BIS-(HYDROXYMETHYL)FURAN (FDM) FROM CRUDE HMF REACTION MIXTURE

The sample of HMF material (30.01 g, 66% HMF) was placed in a 1 L Parr reactor vessel with ethyl acetate (350 mL) and 2.0 g of G-69B. G-69B is a powdered catalyst obtained from Sud-Chemie, Louisville, Kentucky, containing nominally 62% Nickel on Kieselguhr, with a Zirconium promoter, and has an average particle size of 10-14 microns. The vessel was purged 3 X 500 psi hydrogen with vigorous stirring (1000 rpm). The pressure was then maintained at 1250-1050 psi with heating to 150°C for 1 hour. The reaction was allowed to cool and the catalyst removed by filtration. The solvent was removed by rotary evaporation to provide 27.32 g of brown liquid that solidified on cooling. TLC analysis indicated the complete conversion of HMF to FDM. ¹H NMR data reveal a high purity product (>90%). The overall yield of FDM from HMF is 100%. GC/MS data revealed complete conversion of HMF to FDM m/z = 128, 111, 97.

### Reference EXAMPLE 26

### PREPARATION OF 2,5-BIS-(HYDROXYMETHYL)FURAN (FDM) FROM CRUDE HMF REACTION MIXTURE

The sample of HMF material (46.09 g, 45% HMF) was placed in a 1L Parr reactor vessel with ethyl acetate (350 mL) and 6.15 g of G-69B. The vessel was purged 3 x 500 psi hydrogen with vigorous stirring (1000 rpm). The pressure was then maintained at 1350 psi with heating to 150°C for 1 hour. The reaction was allowed to cool and the catalyst removed by filtration. The solvent was removed by rotary evaporation to provide 18.48 g of brown solid. ¹H NMR and gc/ms data reveal a high purity product (>95%). The overall yield of FDM from HMF is 90%. NMR (δ, 1H): 4.54 (s, 2.0 H); 6.20 (s, 1.0 H).

## Claims

1. A method of preparing 2,5-(hydroxymethyl)furaldehyde comprising:
(i) combining a fructose source, a solvent selected from the group consisting of 1-methyl-2-pyrrolidinone, dimethylacetamide, dimethylformamide and combinations of thereof, with a catalyst to provide a reaction mixture;
(ii) heating said reaction mixture to a temperature and for a time sufficient to promote an acid-catalyzed dehydration reaction of fructose in said fructose source to form a product mixture, wherein said acid-catalyzed dehydration reaction is carried out under vacuum and temperature conditions sufficient to remove water from the reaction mixture; and
(iii) isolating 2,5-(hydroxymethyl)furaldehyde from said product mixture.

2. The method of claim 1 wherein said fructose source is comprised of high fructose corn syrup.

3. The method of claim 1 wherein said reaction mixture further includes a water absorbing molecular sieve material.

4. The method of claim 1 wherein said catalyst is selected from the group consisting of a mineral acid, an acidic ion-exchange resin, and a zeolite.

5. The method of claim 1 wherein said catalyst is a solid catalyst comprised of a strong acid ion exchange resin.

6. The method of claim 5 wherein said strong acid ion-exchange resin catalyst is selected from the group consisting of Amberlyst 35, Amberlyst 36, Amberlyst 15, Amberlyst 131, Lewatit S2328, Lewatit K2431, Lewatit S2568, Lewatit K2629, Dianion SK104, Dianion PK228, Dianion RCP160 and Relite RAD/F.

7. The method of claim 1 wherein said reaction mixture is heated to a temperature of from about 95° C to about 125° C.

8. The method of claim 1 wherein said reaction mixture is heated to a temperature of from about 105° C to about 115° C.

9. The method of claim 1 wherein said reaction mixture is heated for a time period of about one to about six hours.

10. The method of claim 1 comprising:
i) combining a fructose source, an organic solvent selected from the group consisting of 1-methyl-2-pyrrolidinone, dimethylacetamide, dimethylformamide and combinations thereof and an acid catalyst to provide a reaction mixture;
ii) heating said reaction mixture to a temperature and for a time sufficient to promote a dehydration reaction of fructose in said fructose source to form a first product mixture, wherein said acid-catalyzed dehydration reaction is carried out under vacuum and temperature conditions sufficient to remove water from the reaction mixture;
iii) neutralizing the pH of the first product mixture to a pH of about 7 to 9;
iv) distilling the first product mixture and neutralizing the pH to remove said organic solvent remaining in the first product mixture; and
v) purifying said product mixture to provide a second product mixture comprising greater than 60% by weight of 2,5-(hydroxymethyl)furaldehyde.

11. The method of claim 10 wherein said fructose source is comprised of high fructose corn syrup.

12. The method of claim 10 wherein said second product mixture comprises greater than 75% by weight of 2,5-(hydroxymethyl)furaldehyde.

13. The method of claim 10 wherein purifying said product isolate comprises a solvent extraction process.

14. The method of claim 13 wherein said solvent extraction process comprises:
(i) adding a mixture comprising a water-immiscible organic solvent and water to said product isolate to provide an organic phase and an aqueous phase;
(ii) recovering said organic phase; and
(iii) removing said water-immiscible solvent from the recovered organic phase to yield purified HMF.

15. The method of claim 14 wherein said water-immiscible organic solvent is selected from the group consisting of ethyl acetate, methyl isobutylketone, methyl ethyl ketone, methyl-t-butyl ether, octanol, pentanol, butyl acetate and combinations thereof.

16. The method of claim 10 wherein the acid catalyst is an acidic ion exchange resin and the acidic ion exchange resin is removed from the first reaction mixture prior to the acid of distilling.

17. The method of claim 10 further comprising adding a non-volatile flowing agent to first product mixture prior to the act of distilling.

18. The method according to claim 17 wherein said non-volatile flowing agent is selected from the group consisting of polyethylene glycol, polyethylene glycol monoether, polyethylene glycol diether, end blocked derivates of polyethylene glycol, polyethylene glycol monoether, polyethylene glycol diether and combinations thereof.

19. The method of claim 10 wherein purifying comprises a process selected from the group consisting of short path distillation, thin film evaporation, wiped film evaporation and adsorption to an inert adsorbent.

20. The method of claim 1 comprising the steps of:
i) combining a fructose source, an acid catalyst, a first organic solvent selected from the group consisting of 1-methyl-2-pyrrolidinone, dimethylacetamide, dimethylformamide and combinations thereof, and a second organic solvent that is non miscible with the first organic solvent to provide a reaction mixture, the first and second organic solvents being selected so that the second organic solvent preferentially dissolves 2,5-(hydroxymethyl)furaldehyde relative to the first organic solvent;
ii) heating said reaction mixture to a temperature and for a time sufficient to promote a dehydration reaction of fructose in said fructose source to form a product mixture with a first immiscible phase and a second immiscible phase, wherein said acid-catalyzed dehydration reaction is carried out under vacuum and temperature conditions sufficient to remove water from the reaction mixture; and
iii) isolating 2,5-(hydroxymethyl)furaldehyde from said second immiscible phase of said product mixture.

21. The method of claim 20 wherein said second organic solvent is selected from the group consisting of methyl isobutyl ketone, ethyl acetate and chloroform.

22. The method of claim 20 wherein said first organic solvent is dimethyl formamide.

23. The method of claim 1 wherein said second organic solvent has a Log P value of from about 0.4 to about 3.4, and said first organic solvent has a Log P value of less than zero.

24. The method of claim 1 wherein said second organic solvent has a Log P value of from about 0.6 to about 2.7.

25. The method of claim 1 wherein said second organic solvent has a Log P value of from -0.075 to about -1.95.

## Patentansprüche

1. Verfahren zur Herstellung von 2,5-(Hydroxymethyl)furaldehyd, umfassend:
(i) Kombinieren einer Fructosequelle, eines Lösungsmittels, das aus der Gruppe ausgewählt ist, die aus 1-Methyl-2-pyrrolidinon, Dimethylacetamid, Dimethylformamid und Kombinationen davon besteht, mit einem Katalysator unter Bildung eines Reaktionsgemischs;
(ii) Erhitzen des Reaktionsgemischs auf eine ausreichende Temperatur und während einer ausreichenden Zeit, um eine säurekatalysierte Dehydratationsreaktion von Fructose in der Fructosequelle voranzutreiben, unter Bildung eines Produktgemischs, wobei die säurekatalysierte Dehydratationsreaktion unter ausreichenden Vakuum- und Temperaturbedingungen durchgeführt wird, um Wasser aus dem Reaktionsgemisch zu entfernen; und
(iii) Isolieren von 2,5-(Hydroxymethyl)furaldehyd aus dem Produktgemisch.

2. Verfahren gemäß Anspruch 1, wobei die Fructosequelle aus fructosereichem Maissirup besteht.

3. Verfahren gemäß Anspruch 1, wobei das Reaktionsgemisch weiterhin ein wasserabsorbierendes Molekularsiebmaterial umfasst.

4. Verfahren gemäß Anspruch 1, wobei der Katalysator aus der Gruppe ausgewählt ist, die aus einer Mineralsäure, einem sauren Ionenaustauscherharz und einem Zeolithen besteht.

5. Verfahren gemäß Anspruch 1, wobei der Katalysator ein fester Katalysator ist, der aus einem stark sauren Ionenaustauscherharz besteht.

6. Verfahren gemäß Anspruch 5, wobei der Katalysator in Form des stark sauren Ionenaustauscherharzes aus der Gruppe ausgewählt ist, die aus Amberlyst 35, Amberlyst 36, Amberlyst 15, Amberlyst 131, Lewatit S2328, Lewatit K2431, Lewatit S2568, Lewatit K2629, Dianion SK104, Dianion PK228, Dianion RCP160 und Relite RAD/F besteht.

7. Verfahren gemäß Anspruch 1, wobei das Reaktionsgemisch auf eine Temperatur von etwa 95 °C bis etwa 125 °C erhitzt wird.

8. Verfahren gemäß Anspruch 1, wobei das Reaktionsgemisch auf eine Temperatur von etwa 105 °C bis etwa 115 °C erhitzt wird.

9. Verfahren gemäß Anspruch 1, wobei das Reaktionsgemisch während einer Zeit von etwa einer bis etwa sechs Stunden erhitzt wird.

10. Verfahren gemäß Anspruch 1, umfassend:
i) Kombinieren einer Fructosequelle, eines organischen Lösungsmittels, das aus der Gruppe ausgewählt ist, die aus 1-Methyl-2-pyrrolidinon, Dimethylacetamid, Dimethylformamid und Kombinationen davon besteht, und eines Säurekatalysators unter Bildung eines Reaktionsgemischs;
ii) Erhitzen des Reaktionsgemischs auf eine ausreichende Temperatur und während einer ausreichenden Zeit, um eine Dehydratationsreaktion von Fructose in der Fructosequelle voranzutreiben, unter Bildung eines ersten Produktgemischs, wobei die säurekatalysierte Dehydratationsreaktion unter ausreichenden Vakuum- und Temperaturbedingungen durchgeführt wird, um Wasser aus dem Reaktionsgemisch zu entfernen;
iii) Neutralisieren des pH-Werts des ersten Produktgemischs auf einen pH-Wert von etwa 7 bis 9;
iv) Destillieren des ersten Produktgemischs und Neutralisieren des pH-Werts, um das im ersten Produktgemisch verbliebene organische Lösungsmittel zu entfernen; und
v) Reinigen des Produktgemischs unter Bildung eines zweiten Produktgemischs, das mehr als 60 Gew.-% 2,5-(Hydroxymethyl)furaldehyd umfasst.

11. Verfahren gemäß Anspruch 10, wobei die Fructosequelle aus fructosereichem Maissirup besteht.

12. Verfahren gemäß Anspruch 10, wobei das zweite Produktgemisch mehr als 75 Gew.-% 2,5-(Hydroxymethyl)furaldehyd umfasst.

13. Verfahren gemäß Anspruch 10, wobei das Reinigen des Produktisolats ein Lösungsmittelextraktionsverfahren umfasst.

14. Verfahren gemäß Anspruch 13, wobei das Lösungsmittelextraktionsverfahren Folgendes umfasst:
(i) Hinzufügen eines Gemischs, das ein mit Wasser nicht mischbares organisches Lösungsmittel und Wasser umfasst, zu dem Produktisolat, wobei man eine organische Phase und eine wässrige Phase erhält;
(ii) Gewinnen der organischen Phase; und
(iii) Entfernen des mit Wasser nicht mischbaren Lösungsmittels aus der gewonnenen organischen Phase, was gereinigtes HMF ergibt.

15. Verfahren gemäß Anspruch 14, wobei das mit Wasser nicht mischbare organische Lösungsmittel aus der Gruppe ausgewählt ist, die aus Ethylacetat, Methylisobutylketon, Methylethylketon, Methyl-t-butylether, Octanol, Pentanol, Butylacetat und Kombinationen davon besteht.

16. Verfahren gemäß Anspruch 10, wobei der saure Katalysator ein saures Ionenaustauscherharz ist und das saure Ionenaustauscherharz vor dem Vorgang des Destillierens aus dem ersten Reaktionsgemisch entfernt wird.

17. Verfahren gemäß Anspruch 10, weiterhin umfassend das Hinzufügen eines nichtflüchtigen Fließmittels zu dem ersten Produktgemisch vor dem Vorgang des Destillierens.

18. Verfahren gemäß Anspruch 17, wobei das nichtflüchtige Fließmittel aus der Gruppe ausgewählt ist, die aus Polyethylenglycol, Polyethylenglycolmonoether, Polyethylenglycoldiether, endblockierten Derivaten von Polyethylenglycol, Polyethylenglycolmonoether, Polyethylenglycoldiether und Kombinationen davon besteht.

19. Verfahren gemäß Anspruch 10, wobei das Reinigen ein Verfahren umfasst, das aus der Gruppe ausgewählt ist, die aus Kurzwegdestillation, Dünnschichtverdampfung, Dünnschichtverdampfung mit Verteilerbürsten und Adsorption an ein inertes Adsorbens besteht.

20. Verfahren gemäß Anspruch 1, umfassend die Schritte:
i) Kombinieren einer Fructosequelle, eines sauren Katalysators, eines ersten organischen Lösungsmittels, das aus der Gruppe ausgewählt ist, die aus 1-Methyl-2-pyrrolidinon, Dimethylacetamid, Dimethylformamid und Kombinationen davon besteht, und eines zweiten organischen Lösungsmittels, das mit dem ersten organischen Lösungsmittel nicht mischbar ist, unter Bildung eines Reaktionsgemischs, wobei das erste und das zweite organische Lösungsmittel so ausgewählt sind, dass das zweite organische Lösungsmittel 2,5-(Hydroxymethyl)furaldehyd besser auflöst als das erste organische Lösungsmittel;
ii) Erhitzen des Reaktionsgemischs auf eine ausreichende Temperatur und während einer ausreichenden Zeit, um eine Dehydratationsreaktion von Fructose in der Fructosequelle voranzutreiben, unter Bildung eines Produktgemischs mit einer ersten unmischbaren Phase und einer zweiten unmischbaren Phase, wobei die säurekatalysierte Dehydratationsreaktion unter ausreichenden Vakuum- und Temperaturbedingungen durchgeführt wird, um Wasser aus dem Reaktionsgemisch zu entfernen; und
iii) Isolieren von 2,5-(Hydroxymethyl)furaldehyd aus der zweiten unmischbaren Phase des Produktgemischs.

21. Verfahren gemäß Anspruch 20, wobei das zweite organische Lösungsmittel aus der Gruppe ausgewählt ist, die aus Methylisobutylketon, Ethylacetat und Chloroform besteht.

22. Verfahren gemäß Anspruch 20, wobei es sich bei dem ersten organischen Lösungsmittel um Dimethylformamid handelt.

23. Verfahren gemäß Anspruch 1, wobei das zweite organische Lösungsmittel einen Log-P-Wert von etwa 0,4 bis etwa 3,4 aufweist und das erste organische Lösungsmittel einen Log-P-Wert von weniger als null aufweist.

24. Verfahren gemäß Anspruch 1, wobei das zweite organische Lösungsmittel einen Log-P-Wert von etwa 0,6 bis etwa 2,7 aufweist.

25. Verfahren gemäß Anspruch 1, wobei das zweite organische Lösungsmittel einen Log-P-Wert von -0,075 bis etwa -1,95 aufweist.

## Revendications

1. Procédé de préparation de 2,5-(hydroxyméthyl)furaldéhyde comprenant :
(i) la combinaison d'une source de fructose, d'un solvant choisi dans le groupe consistant en la 1-méthyl-2-pyrrolidinone, le diméthylacétamide, le diméthylformamide et des combinaisons de ceux-ci, avec un catalyseur pour fournir un mélange de réaction ;
(ii) le chauffage dudit mélange de réaction à une température et pendant un temps suffisants pour promouvoir une réaction de déshydratation catalysée par acide du fructose dans ladite source de fructose afin de former un mélange de produit, où ladite réaction de déshydratation catalysée par acide est réalisée sous vide et dans des conditions de température suffisantes pour éliminer l'eau du mélange de réaction ; et
(iii) l'isolement du 2,5-(hydroxyméthyl)furaldéhyde dudit mélange de produit.

2. Procédé selon la revendication 1, dans lequel ladite source de fructose est composée d'un sirop de maïs à teneur élevée en fructose.

3. Procédé selon la revendication 1, dans lequel ledit mélange de réaction comprend en outre un matériau de tamis moléculaire absorbant l'eau.

4. Procédé selon la revendication 1, dans lequel ledit catalyseur est choisi dans le groupe consistant en un acide minéral, une résine échangeuse d'ions acide et une zéolithe.

5. Procédé selon la revendication 1, dans lequel ledit catalyseur est un catalyseur solide composé d'une résine échangeuse d'ions à acide fort.

6. Procédé selon la revendication 5, dans lequel ledit catalyseur de résine échangeuse d'ions à acide fort est choisi dans le groupe consistant en Amberlyst 35, Amberlyst 36, Amberlyst 15, Amberlyst 131, Lewatit S2328, Lewatit K2431, Lewatit S2568, Lewatit K2629, Dianion SK104, Dianion PK228, Dianion RCP160 et Relite RAD/F.

7. Procédé selon la revendication 1, dans lequel ledit mélange de réaction est chauffé à une température d'environ 95 °C à environ 125 °C.

8. Procédé selon la revendication 1, dans lequel ledit mélange de réaction est chauffé à une température d'environ 105 °C à environ 115 °C.

9. Procédé selon la revendication 1, dans lequel ledit mélange de réaction est chauffé pendant une période de temps d'environ une à environ six heures.

10. Procédé selon la revendication 1, comprenant :
i) la combinaison d'une source de fructose, d'un solvant organique choisi dans le groupe consistant en la 1-méthyl-2-pyrrolidinone, le diméthylacétamide, le diméthylformamide et des combinaisons de ceux-ci, et d'un catalyseur acide pour fournir un mélange de réaction ;
ii) le chauffage dudit mélange de réaction à une température et pendant un temps suffisants pour promouvoir une réaction de déshydratation du fructose dans ladite source de fructose afin de former un premier mélange de produit, où ladite réaction de déshydratation catalysée par acide est réalisée sous vide et dans des conditions de température suffisantes pour éliminer l'eau du mélange de réaction ;
iii) la neutralisation du pH du premier mélange de produit à un pH d'environ 7 à 9 ;
iv) la distillation du premier mélange de produit et la neutralisation du pH afin d'éliminer ledit solvant organique restant dans le premier mélange de produit ; et
v) la purification dudit mélange de produit afin de fournir un second mélange de produit comprenant plus de 60 % en poids de 2,5-(hydroxyméthyl)furaldéhyde.

11. Procédé selon la revendication 10, dans lequel ladite source de fructose est composée de sirop de maïs à teneur élevée en fructose.

12. Procédé selon la revendication 10, dans lequel ledit second mélange de produit comprend plus de 75 % en poids de 2,5-(hydroxyméthyl)furaldéhyde.

13. Procédé selon la revendication 10, dans lequel la purification dudit isolat de produit comprend un processus d'extraction de solvant.

14. Procédé selon la revendication 13, dans lequel ledit processus d'extraction de solvant comprend :
(i) l'ajout d'un mélange comprenant un solvant organique non miscible dans l'eau et de l'eau audit isolat de produit pour fournir une phase organique et une phase aqueuse ;
(ii) la récupération de ladite phase organique ; et
(iii) l'élimination dudit solvant non miscible dans l'eau à partir de la phase organique récupérée afin d'obtenir du HMF purifié.

15. Procédé selon la revendication 14, dans lequel ledit solvant organique non miscible dans l'eau est choisi dans le groupe consistant en l'acétate d'éthyle, la méthylisobutylcétone, la méthyléthylcétone, le méthyl-t-butyléther, l'octanol, le pentanol, l'acétate de butyle et des combinaisons de ceux-ci.

16. Procédé selon la revendication 10, dans lequel le catalyseur acide est une résine échangeuse d'ions acide et la résine échangeuse d'ions acide est éliminée du premier mélange de réaction avant l'acte de distillation.

17. Procédé selon la revendication 10, comprenant en outre l'ajout d'un agent fluidifiant non volatil au premier mélange de produit avant l'acte de distillation.

18. Procédé selon la revendication 17, dans lequel ledit agent fluidifiant non volatil est choisi dans le groupe consistant en le poly(éthylène glycol), le poly(éthylène glycol)monoéther, le poly(éthylène glycol)diéther, des dérivés à extrémité séquencée de poly(éthylène glycol), de poly(éthylène glycol)monoéther, de poly(éthylène glycol)diéther et des combinaisons de ceux-ci.

19. Procédé selon la revendication 10, dans lequel la purification comprend un processus choisi dans le groupe consistant en une distillation à court trajet, une évaporation en couche mince, une évaporation à couche raclée et une adsorption sur un adsorbant inerte.

20. Procédé selon la revendication 1, comprenant les étapes de :
i) combinaison d'une source de fructose, d'un catalyseur acide, d'un premier solvant organique choisi dans le groupe consistant en la 1-méthyl-2-pyrrolidinone, le diméthylacétamide, le diméthylformamide et des combinaisons de ceux-ci, et d'un second solvant organique qui est non miscible avec le premier solvant organique pour fournir un mélange de réaction,
les premier et second solvants organiques étant choisis de sorte que le second solvant organique dissolve de préférence le 2,5-(hydroxyméthyl)furaldéhyde par rapport au premier solvant organique ;
ii) chauffage dudit mélange de réaction à une température et pendant un temps suffisants pour promouvoir une réaction de déshydratation de fructose dans ladite source de fructose afin de former un mélange de produit avec une première phase non miscible et une seconde phase non miscible, où ladite réaction de déshydratation catalysée par acide est réalisée sous vide et dans des conditions de température suffisantes pour éliminer l'eau du mélange de réaction ; et
(iii) isolement du 2,5-(hydroxyméthyl)furaldéhyde de ladite seconde phase non miscible dudit mélange de produit.

21. Procédé selon la revendication 20, dans lequel ledit second solvant organique est choisi dans le groupe consistant en la méthylisobutylcétone, l'acétate d'éthyle et le chloroforme.

22. Procédé selon la revendication 20, dans lequel ledit premier solvant organique est le diméthylformamide.

23. Procédé selon la revendication 1, dans lequel ledit second solvant organique a une valeur de Log P d'environ 0,4 à environ 3,4, et ledit premier solvant organique a une valeur de Log P inférieure à zéro.

24. Procédé selon la revendication 1, dans lequel ledit second solvant organique a une valeur de Log P d'environ 0,6 à environ 2,7.

25. Procédé selon la revendication 1, dans lequel ledit second solvant organique a une valeur de Log P de -0,075 à environ -1,95.
